(19) Europäisches Patentamt
European Patent Office
Office européen des brevets

(11) Veröffentlichungsnummer: **0 039 059**
**B1**

(12) # EUROPÄISCHE PATENTSCHRIFT

(45) Veröffentlichungstag der Patentschrift: **25.06.86**

(21) Anmeldenummer: **81103103.8**

(22) Anmeldetag: **24.04.81**

(51) Int. Cl.⁴: **A 61 K 31/645,** A 61 K 31/495, A 61 K 31/55 // (A61K31/495, 31:415),(A61K31/55, 31:415)

(54) **Entzündungshemmende Mittel und deren Verwendung.**

(30) Priorität: **25.04.80 US 143699**

(43) Veröffentlichungstag der Anmeldung: **04.11.81 Patentblatt 81/44**

(45) Bekanntmachung des Hinweises auf die Patenterteilung: **25.06.86 Patentblatt 86/26**

(84) Benannte Vertragsstaaten: **CH DE FR GB IT LI LU NL SE**

(56) Entgegenhaltungen:
**DE - A - 1 670 032**
**DE - A - 1 801 523**
**DE - A - 2 252 806**
**US - A - 3 681 354**
**US - A - 4 034 087**
**US - A - 4 049 809**

**SUCKER H. et al., "Pharmazeutische Technologie" Georg Thieme Verlag Stuttgart (1978), S. 630**

(73) Patentinhaber: **F. HOFFMANN-LA ROCHE & CO. Aktiengesellschaft, CH-4002 Basel (CH)**

(72) Erfinder: **Boris, Alfred, 25 Lord Sterling Drive, Parsippany-Troy Hills, N.J. 07054 (US)**

(74) Vertreter: **Lederer, Franz, Dr. et al, Patentanwälte Dr. Franz Lederer Dipl.-Ing. Reiner F. Meyer-Roxlau Lucile-Grahn-Strasse 22, D-8000 München 80 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

**Beschreibung**

Die vorliegende Erfindung betrifft topische Arzneimittel. Unter den bekannteren Krankheiten, die mit den erfindungsgemässen Arzneimitteln topisch behandelt werden können, sind Psoriasis, Eczemata, Dermatitis verschiedener Ätiologie und Keratosis zu erwähnen. Diese Aufzählung dient lediglich der Illustration.

Im speziellen betrifft die vorliegende Erfindung Arzneimittel für die topische Anwendung auf entzündeten Flächen. Die in der Vergangenheit für die topische Behandlung von Entzündungen meist verwendeten Wirkstoffe waren die Corticosteroide, d.h. von der Nebennierenrinde ausgeschiedene Steroide, und ihre synthetischen Analoga, insbesondere Hydrocortison. Wenn auch die Corticosteroide Entzündungen und verwandte Symptome wirksam zu bekämpfen vermögen, so zeigen sie doch unerwünschte Nebenwirkungen, insbesondere wenn sie über einen längeren Zeitraum angewendet werden. Unter den Nebenwirkungen, welche bei längerfristiger Anwendung von Corticosteroiden auftreten können, sind Dünnerwerden und Streifenbildung der Haut und Störungen des körpereigenen immunologischen Systems zu erwähnen. Diese unerwünschten Nebenwirkungen waren der Anlass für die Forschung, nach pharmakologisch wirksamen Verbindungen zu suchen, welche nicht der Klasse der Steroide angehören und vergleichbare topisch entzündungshemmende Wirkungen besitzen wie die Corticosteroide und welche keine begleitenden unerwünschten Nebenwirkungen zeigen.

Es wurden eine ganze Anzahl von Verbindungen gefunden, welche systemische entzündungshemmende Wirkungen besitzen und nicht zur Klasse der Steroide gehören. Als Beispiele seien Phenylbutazon, Naproxin und Ibuprofen genannt. Es hat sich jedoch gezeigt, dass Verbindungen, welche systemische entzündungshemmende Wirkungen besitzen und nicht zur Klasse der Steroide gehören, nicht nützlich sind für die topische Behandlung von Hautentzündungen. Wenn auch die Ätiologie von Hautentzündungen noch nicht ganz verstanden wird, so nimmt man doch im allgemeinen an, dass sie stark verschieden ist von der Ätiologie von systemischen Entzündungen. Darüberhinaus unterliegt die Absorption von Verbindungen durch die zahlreichen Hautschichten ganz anderen physikochemischen Bedingungen als die Absorptions- und Verteilungsprozesse auf systemischer Basis. Aus diesen Gründen kann man nicht erwarten, dass ein systemisch wirksames, entzündungshemmendes Mittel auch topisch wirksam ist, insbesondere wenn es sich um Wirkstoffe handelt, die nicht zur Klasse der Steroide gehören.

Erfindungsgemäss wurde gefunden, dass Entzündungen der Hautgewebe behandelt werden können, indem man eine wirksame Menge einer Verbindung der allgemeinen Formel

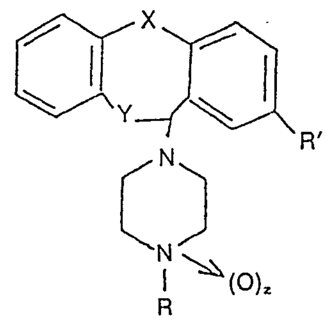

I

worin X ein Schwefel- oder Sauerstoffatom, Y die Gruppe $-CH=$, $-CH_2-$ oder $-N=$, R niederes Alkyl oder Hydroxy-niederes Alkyl, R′ niederes Alkyl, Halogen, Cyano, niederes Alkylthio, Carboxy oder niederes Alkylcarboxy und Z die Zahl 0 oder 1 bedeuten, mit der Massgabe, dass R′ Brom und X ein Schwefelatom bedeuten, wenn Y die Gruppe $-CH_2-$ bedeutet, und X ein Sauerstoffatom bedeutet, wenn Y die Gruppe $-N=$ bedeutet, oder eines pharmazeutisch annehmbaren Salzes davon topisch anwendet.

Die vorliegende Erfindung betrifft somit entzündungshemmende Mittel für topische, d.h. für lokale äusserliche Anwendung, enthaltend eine Verbindung der allgemeinen Formel I oder ein pharmazeutisch annehmbares Salz davon und ein für die topische, d.h. für die lokale äusserliche Anwendung bestimmtes pharmazeutisch annehmbares Trägermaterial.

Erfindungsgemäss wurde auch gefunden, dass die Wirkung einer Verbindung der allgemeinen Formel I als topisch entzündungshemmendes Mittel durch die Verwendung in Kombination mit einer Verbindung der allgemeinen Formel

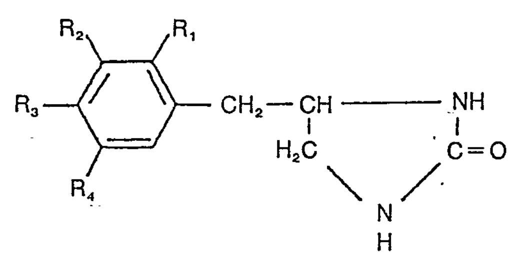

II

worin $R_1$ Halogen, Wasserstoff, niederes Alkyl oder niederes Alkoxy und $R_2$, $R_3$ und $R_4$ unabhängig voneinander Wasserstoff, niederes Alkoxy oder Hydroxy-niederes Alkoxy bedeuten, mit der Massgabe, dass mindestens einer der Reste $R_2$, $R_3$ und $R_4$ einen sauerstoffhaltigen Substituenten bedeutet, oder je zwei benachbarte Reste $R_1$, $R_2$, $R_3$ und $R_4$ zusammen Methylendioxy bedeuten, signifikant gesteigert werden kann.

Dementsprechend können erwünschtenfalls eine oder mehrere Verbindungen der allgemeinen Formel II in den erfindungsgemässen topischen Arzneimitteln enthalten sein.

Arzneimittel, enthaltend eine Verbindung der allgemeinen Formel I oder ein pharmazeutisch annehmbares Salz davon und eine Verbindung der allgemeinen Formel II sind per se neu, d.h. unabhängig von der Art der galenischen Darreichungsform, welche diese Verbindungen enthält, und sind ebenfalls Gegenstand der vorliegenden Erfindung.

Die vorliegende Erfindung betrifft ausserdem die Verwendung von Verbindungen der allgemeinen Formel I oder von pharmazeutisch annehmbaren Salzen davon, gegebenenfalls in Kombination mit einer Verbindung der allgemeinen Formel II, für die Herstellung von Arzneimitteln zur lokalen äusserlichen Behandlung von Entzündungen der Hautgewebe, wie Psoriasis ganz allgemein, Eczemata, Dermatitis verschiedener Ätiologie, Keratosis und dergleichen.

Der in der vorliegenden Beschreibung verwendete Ausdruck «niederes Alkyl» bezeichnet geradkettige oder verzweigte gesättigte Kohlenwasserstoffreste mit 1–7, vorzugsweise 1–4, Kohlenstoffatomen. Beispiele für niederes Alkyl sind Methyl, Äthyl, n-Propyl, Isopropyl, n-Propyl, n-Butyl, n-Hexyl und n-Heptyl. Der Ausdruck «Halogen» umfasst Fluor, Chlor, Brom und Jod.

Als Beispiele für Verbindungen der allgemeinen Formel I seien die folgenden genannt:

8-Chlor-10-(4-methylpiperazino)dibenzo[b,f]-thiepin,
8-Brom-10-(4-methylpiperazino)dibenzo[b,f]-thiepin,
8-Methylthio-10-(4-methylpiperazino)dibenzo-[b,f]thiepin,
8-Cyano-10-(4-methylpiperazino)-dibenzo[b,f]-thiepin,
8-Methylthio-10-(4-methylpiperazino)-dibenzo-[b,f]thiepin-$N^4$-oxid,
8-Cyano-10-(4-methylpiperazino)-dibenzo[b,f]-thiepin-$N^4$-oxid,
4-(8-Chlordibenzo[b,f]thiepin-10-yl)-1-piperazin-propanol,
die entsprechenden Oxepine,
8-Brom-10-(4-methylpiperazino)-10,11-dihydrodibenzo[b,f]thiepin und
2-Chlor-11-(4-methyl-1-piperazinyl)dibenzo-[b,f]4,1]oxazepin.

Bevorzugt werden Verbindungen der allgemeinen Formel I, worin X ein Schwefelatom, Y die Gruppe –CH=, R Methyl und Z die Zahl 0 bedeuten. Eine ganz besonders bevorzugte Verbindung der allgemeinen Formel I ist
8-Chlor-10-(4-methylpiperazino)dibenzo[b,f]-thiepin.

Die Verbindungen der allgemeinen Formel I sind bekannt oder können nach an sich bekannten Verfahren hergestellt werden. Sie können beispielsweise nach Verfahren hergestellt werden, welche in der U.S. Patentschrift 3 681 354, in der Deutschen Offenlegungsschrift 22 52 806, in Collect. Czech. Chem. Comm. 35, 3721–3723 (1970) und in der U.S. Patentschrift 3 546 226 beschrieben sind.

Die U.S. Patentschrift 3 681 354 beschreibt die Herstellung von Verbindungen der allgemeinen Formel I, worin R′ niederes Alkyl oder Halogen, Y die Gruppe –CH= und Z die Zahl 0 bedeuten. Diese Patentschrift offenbart, dass diese Verbindungen unter anderem entzündungshemmende Eigenschaften besitzen, allerdings nur wenn sie oral verabreicht werden, d.h. dass diese Verbindungen systemisch entzündungshemmend wirken. Dass diese Verbindungen topisch entzündungshemmende Eigenschaften besitzen, konnte nicht erwartet werden und ist völlig überraschend.

Die Deutsche Offenlegungsschrift 22 52 806 beschreibt die Herstellung von Verbindungen der allgemeinen Formel I, worin R′ Cyano, Halogen oder Alkylthio, X ein Schwefelatom und Y die Gruppe –CH= bedeuten. Die entsprechenden Verbindungen, worin X ein Sauerstoffatom bedeutet, können in analoger Weise hergestellt werden. Verbindungen der allgemeinen Formel I, worin Z die Zahl 1 bedeutet, können ebenfalls nach Verfahren hergestellt werden, welche in der Deutschen Offenlegungsschrift 22 52 806 beschrieben sind. Gemäss dieser Offenlegungsschrift besitzen diese Verbindungen psychodepressive Wirkungen.

Collect. Czech. Chem., Comm. 35, 3721–3723 (1970), beschreibt die Herstellung von Verbindungen der allgemeinen Formel I, worin R′ niederes Alkylthio bedeutet. Diese Verbindungen werden darin als Neuroleptika offenbart.

Die U.S. Patentschrift 3 546 226 beschreibt die Herstellung von Verbindungen der allgemeinen Formel I, worin X ein Sauerstoffatom und Y die Gruppe –N= bedeuten, d.h. Oxazepine. Diese Verbindungen werden darin als Neuroleptika offenbart.

Die Verbindungen der allgemeinen Formel I bilden Säureadditionssalze mit herkömmlichen pharmazeutisch annehmbaren Säuren, d.h. mit anorganischen Säuren, wie Salzsäure, Schwefelsäure, Phosphorsäure, Bromwasserstoffsäure und dergleichen, oder mit organischen Säuren, wie Zitronensäure, Essigsäure, Bernsteinsäure, Maleinsäure, Methansulfonsäure, p-Toluolsulfonsäure und dergleichen.

Die Verbindungen der allgemeinen Formel I oder ihre Salze werden in Form von pharmazeutischen Präparaten verwendet, welche eine wirksame Menge einer Verbindung der Formel I oder eines pharmazeutisch annehmbaren Salzes davon und ein pharmazeutisch annehmbares, für die topische Anwendung bestimmtes Trägermaterial enthalten. Erfindungsgemässe topische Dosierungsformen enthalten, bezogen auf ihr Gesamtgewicht, in der Regel etwa 0,01 bis 10 Gewichtsprozent, vorzugsweise 0,1 bis 1,0 Gewichtsprozent, einer Verbindung der allgemeinen Formel I oder eines pharmazeutisch annehmbaren Salzes davon. Die minimale wirksame Menge einer Verbindung der allgemeinen Formel I oder eines pharmazeutisch annehmbaren Salzes davon kann in gewissen Grenzen variieren und hängt davon ab, welche Verbindung der allgemeinen Formel I oder welches pharmazeutisch annehmbare Salz davon verwendet wird. Immerhin bereitet es dem behandelnden Arzt keine Schwierigkeiten, in jedem einzelnen Fall die wirksame Dosis zu bestimmen. Unter «wirksame Dosis» versteht man diejenige Menge, welche notwendig ist, um die Symptome der Entzündung zu reduzieren; z.B. die Rötung, Schuppenbildung, Juckreiz und Schmerzen zu vermindern oder die Hautverdickung an der entzündeten Stelle zu reduzieren. Üblicherweise werden die topischen Präparate 1 bis 6 mal täglich in dünnen Schichten auf die entzündeten Flächen aufgetragen. Die Behandlung hängt natürlich von verschiedenen Faktoren ab, z.B. von der Stärke und von der Art der zu behandelnden Krankheit, von der Lokalisierung der zu behandelnden entzündeten Fläche und von der Konzentration des aktiven Wirkstoffes im topischen Präparat.

Wie bereits weiter oben erwähnt können die erfindungsgemässen topischen pharmazeutischen Präparate zusätzlich zu den Verbindungen der allgemeinen Formel I oder ihren pharmazeutisch annehmbaren Salzen eine oder mehrere Verbindungen der allgemeinen Formel II enthalten.

Solche Kombinationspräparate können in der Regel, bezogen auf ihr Gesamtgewicht, etwa 0,1 bis 10 Gewichtsprozent, vorzugsweise 0,5 bis 5 Gewichtsprozent, einer Verbindung der allgemeinen Formel II enthalten. Das geeignete Verhältnis einer Verbindung der allgemeinen Formel II zu einer Verbindung der allgemeinen Formel I liegt zweckmässigerweise in einem Bereich von etwa 2:1 bis etwa 100:1, vorzugsweise von 10:1 bis 50:1.

Die Verbindungen der allgemeinen Formel II und Verfahren zu ihrer Herstellung sind bekannt. Obwohl es für die Dosierung der Verbindung der allgemeinen Formel II in einem erfindungsgemässen topischen pharmazeutischen Präparat keine genaue obere Grenze gibt, erreicht man keine besonderen Vorteile, wenn, bezogen auf das Gesamtgewicht des Präparates, mehr als 10 Gewichtsprozent vorhanden sind. Eine bevorzugte Verbindung der allgemeinen Formel II ist
4-(3-Butoxy-4-methoxybenzyl)-2-imidazolidinon.

Verwendet man eine Verbindung der allgemeinen Formel II in einem erfindungsgemässen topischen pharmazeutischen Präparat, so kann die Dosierung einer Verbindung der Formel I oder eines pharmazeutisch annehmbaren Salzes davon vermindert werden, ohne dass die entzündungshemmende Wirksamkeit nachlässt. Es war nicht vorauszusehen, dass eine Verbindung der allgemeinen Formel II die Wirksamkeit eines topisch entzündungshemmenden Wirkstoffes der allgemeinen Formel I oder eines pharmazeutisch annehmbaren Salzes davon steigern würde, da
4-(3-Butoxy-4-methoxybenzyl)-2-imidazolidinon,
d.h. eine Verbindung der Formel II, bei Konzentrationen von 5% selber nur wenig oder gar keine entzündungshemmende Wirkung zeigt.

Der Ausdruck «topisch», wie er in der vorliegenden Beschreibung verwendet wird, bezieht sich auf die Verwendung des aktiven Bestandteils, das mit einem geeigneten Trägermaterial verarbeitet und äusserlich auf die entzündete Stelle gebracht wird, so dass es die lokale Wirkung entfalten kann. Demgemäss umfassen die topischen Arzneimittel für die äusserliche Anwendung bestimmte, pharmazeutische Dosierungsformen, so dass ein direkter Kontakt mit der Haut entsteht. Die topischen Dosierungsformen umfassen Gele, Crèmes, Lotionen, Salben, Puders, Aerosole und andere herkömmliche Formen, welche für die direkte Applikation von Arzneimitteln auf die Haut geeignet sind. Diese Dosierungsformen können hergestellt werden, indem man Verbindungen der allgemeinen Formel I oder pharmazeutisch annehmbare Salze davon und gegebenenfalls Verbindungen der allgemeinen Formel II mit bekannten pharmazeutischen, für die topische Anwendung geeigneten Trägermaterialien vermischt.

Salben und Crèmes enthalten ölige, absorbierende, wasserlösliche oder emulgierende Trägermaterialien, wie Paraffin, Lanolin, Polyäthylenglykole und dergleichen.

Lotionen sind flüssige Präparate und können wässrige oder wässrig/alkoholische Präparate, welche die Substanzen in fein verteilter Form enthalten, sein. Die Präparate enthalten suspendierende oder dispergierende Stoffe, wie Cellulosederivate (z.B. Äthylcellulose, Methylcellulose, etc), Gelatine oder Gummi arabicum, welche den Wirkstoff in einem aus Wasser, Alkohol, Glyzerin, hergestellten Träger suspendieren bzw. dispergieren.

Gele sind halbfeste Präparate, welche man durch Gelieren einer Lösung oder Suspension des Wirkstoffes in einem Trägermaterial enthält. Die Trägermaterialien, welche wässrig oder nicht wässrig sein können, werden unter Verwendung eines Geliermittels, wie Carboxypolymethylen, geliert und unter Verwendung von Alkalien, wie Natriumhydroxid, und Aminen, wie Polyäthylencocoamin, zu einem Gel geeigneter Konsistenz neutralisiert.

Aerosole sind Lösungen oder Suspensionen des Wirkstoffes in einem inerten Trägermaterial, welche unter Verwendung von speziellen Sprüheinrichtungen verteilt werden. Üblicherweise verwendete Träger sind Trichlormonofluormethan und Trichlordifluormethan.

Die erfindungsgemässen pharmazeutischen Präparate können herkömmlichen pharmazeutischen Nachbehandlungsmassnahmen, wie Sterilisation, unterworfen werden und/oder können herkömmliche pharmazeutische Hilfsstoffe enthalten, wie Konservierungsmittel, Stabilisierungsmittel, Netzmittel, Emulgiermittel, Salze zur Veränderung des osmotischen Druckes, Puffer und dergleichen. Die pharmazeutischen Präparate können erwünschtenfalls auch andere therapeutisch wertvolle Stoffe, andere topisch entzündungshemmende Mittel eingeschlossen, enthalten, und zwar in Kombination mit Verbindungen der allgemeinen Formel I oder pharmazeutisch annehmbaren Salzen davon und, sofern vorhanden, mit Verbindungen der allgemeinen Formel II. Diese Bestandteile werden in ihren bekannten wirksamen Konzentrationen verwendet.

Die folgenden Experimente und Beispiele sollen die vorliegende Erfindung illustrieren. Sofern nichts anderes angegeben wird, handelt es sich bei den Teilen und Prozenten um Gewichtsteile

und Gewichtsprozente. Alle Temperaturen sind in Celsiusgraden angegeben.

In den Experimenten werden die folgenden Testverfahren verwendet, um die topisch entzündungshemmende Wirkung der getesteten Verbindungen zu zeigen.

1. Cantharidin-Entzündung in der Ratte

Eine Lösung aus einem Teil Äthanol, 1,5 Teilen Collodion, 2 Teilen Aceton und 3 Teilen Diäthyläther (Volumenteile), welche 400 µg Cantharidin/ 0,1 ml und die Testverbindung in der gewünschten Menge enthält, wird topisch auf die äussere Oberfläche von Ohren von 21 Tage alten männlichen Ratten (Charles River CD) gegeben (0,1 ml). Andere Gruppen von Ratten werden mit Trägermaterial alleine, mit Cantharidin alleine und mit Cantharidin plus Testverbindung behandelt. Wo in den Beispielen angegeben, wird die Testverbindung 24 Stunden nach Verabreichung von Cantharidin verabreicht. 72 Stunden nach Verabreichung von Cantharidin wird eine Autopsie durchgeführt. Aus dem Ohr werden an der behandelten Stelle einheitliche Stücke herausgestanzt und gewogen. Aus den Gewichtsveränderungen lassen sich die Auswirkungen auf den Entzündungsprozess ermitteln, und zwar sowohl für die Flüssigkeits- als auch für die Gewebskomponente. Eine Reduktion des Gewichtes von Hautstücken, die man aus Ratten erhalten hat, welche mit dem Reizmittel und der Testverbindung behandelt worden sind, verglichen mit dem Gewicht von Hautstücken, welche man von Ratten erhalten hat, die nur mit dem Reizmittel behandelt worden sind, zeigt eine entzündungshemmende Wirkung an (J. Invest. Dermatol. 68, 161–164, 1977).

2. Krotonöl-Entzündung in der Maus

Eine Lösung aus 0,1 Teilen Krotonöl, 1 Teil destilliertem Wasser, 4 Teilen Pyridin und 4,9 Teilen Diäthyläther (Volumenteile), welche die Testverbindung enthält, wird topisch auf Ohren von 21 Tage alten männlichen Mäusen (Charles River CD-1) gegeben (25 µl). Verschiedene Gruppen von Mäusen dienten als Kontrollgruppen (Trägermaterial- Gruppe und Krotonöl-Gruppe). Nach 6 Stunden wird das Gewicht der Ohrstücke bestimmt, das in erster Linie ein Mass für die Ödem-Komponente der Entzündung ist. Verminderung des Gewichtes von Ohrstücken, welche von Mäusen erhalten wurden, welche mit der Testverbindung und dem Reizmittel behandelt worden sind, verglichen mit dem Gewicht von Ohrstücken, die von Mäusen erhalten wurden, die nur mit dem Reizmittel behandelt worden sind, zeigt entzündungshemmende Wirkung an (Endocrinology 77, 625–634, 1965; Clin. Pharm. Exp. Therap. 16, 900–904, 1974).

3. Oxazolon-Entzündung in vorsensibilisierten Mäusen

21 Tage alte männliche Mäuse (Charles River CD-1) werden mit Oxazolon sensibilisiert, indem man 400 µg Oxazolon in 20 µl Aceton topisch auf das Scrotum appliziert. Eine Woche später werden 10 µl Aceton oder 200 µg Oxazolon topisch auf die innere Oberfläche des Ohres und die Testverbindung in 10 µl Aceton auf die äussere Oberfläche des Ohres appliziert. Verschiedene Gruppen von Mäusen dienten als Kontrollgruppen (Trägermaterial-Gruppe und Oxazolon-Gruppe). Das Gewicht der Ohrstücke wird 24 Stunden später bestimmt. Verminderung des Gewichtes der Ohrstücke, erhalten aus Mäusen, welche mit der Testverbindung und mit Oxazolon behandelt worden sind, verglichen mit dem Gewicht der Ohrstücke, erhalten aus Mäusen, welche mit Oxazolon alleine behandelt worden sind, zeigt entzündungshemmende Wirkungen (Br. J. Pharmacol. 43, 403–408, 1971).

4. Baumwollkügelchen-Granuloma-Verfahren an der Ratte

Man löst die Testverbindung in einem geeigneten Lösungsmittel und appliziert jeweils 0,1 ml dieser Lösung auf einheitliche, vorher gewogene Baumwollkügelchen, die man aus Baumwolltampons herausgeschnitten hat. Man trocknet die Baumwollkügelchen bei Raumtemperatur. 2 mit der Testverbindung imprägnierte Kügelchen werden auf dem Dorsum von 5 Wochen alten männlichen Ratten (Charles River CD) subcutan inplantiert. 72 Stunden später werden die Kügelchen entfernt. Man bestimmt das Feucht- und Trockengewicht und korrigiert um das Tara der Kügelchen. Vergleicht man nun die Gewichte der Granulomata, die man von mit der Testverbindung imprägnierten Kügelchen erhalten hat, mit den Gewichten der Granulomata, die man von nur mit Trägermaterial imprägnierten Kügelchen erhalten hat, miteinander, so zeigt eine Abnahme des Gewichtes entzündungshemmende Wirkung an (Endocrinology 60, 153–160, 1957).

In den Experimenten wurden die folgenden Verbindungen als Testverbindungen verwendet:

Verbindung A: 8-Methylthio-10-(4-methylpiperazino)dibenzo [b,f]thiepin,

Verbindung B: 4-(8-Chlordibenzo[b,f]thiepin-10-yl)-1-piperazin-propanol,

Verbindung C: 8-Methylthio-10-(4-methylpiperazino)dibenzo[b,f]thiepin-$N^4$-oxid,

Verbindung D: 8-Chlor-10-(4-methylpiperazino)-dibenzo[b,f]thiepin-$N^4$-oxid,

Verbindung E: 8-Chlor-10-(4-methylpiperazino)-dibenzo[b,f]thiepin,

Verbindung F: 8-Brom-10-(4-methylpiperazino)-10,11-dihydro-dibenzo[b,f]thiepin,

Verbindung G: 8-Cyano-10-(4-methylpiperazino) dibenzo[b,f]thiepin-$N^4$-oxid,

Verbindung H: 8-Cyano-10-(4-methylpiperazino)-dibenzo[b,f]thiepin,

Verbindung I: 8-Brom-10-(4-methylpiperazino)-dibenzo[b,f]thiepin,

Verbindung J: 8-Fluor-10-(4-methylpiperazino)-dibenzo[b,f]thiepin,

Verbindung K: 2-Chlor-11-(4-methyl-1-piperazinyl)dibenz[b,f][1,4]oxazepin und

Verbindung L: 8-Chlor-10-(4-methylpiperazino)-dibenz[b,f]oxepin.

Experiment 1

Eine Reihe von Verbindungen der allgemeinen Formel I werden im Cantharidin-Entzündungs-Test in der Ratte auf ihre topisch entzündungshemmenden Wirkungen hin getestet. Jede Verbindung wird bei 5 verschiedenen Dosierungen getestet. Die in der nachfolgenden Tabelle angegebenen Testresultate (in %) geben an, um wieviel die durch Cantharidin bewirkte Zunahme des Gewichtes der Ohrstücke durch die jeweilige Testverbindung reduziert wird:

Topisch entzündungshemmende Wirkung im Cantharidin-Ratten-Modell

| | Hemmung der Cantharidin-Entzündung in % | | | | |
|---|---|---|---|---|---|
| Verbindung | 50 µg/0.1 ml | 100 µg/0.1 ml | 200 µg/0.1 ml | 400 µg/0.1 ml | 800 µg/0.1 ml |
| A | 33 | 44 | 89 | 101 | 104 |
| B | 0 | 0 | 0 | 57 | 81 |
| C | 0 | 0 | 0 | 31 | 82 |
| D | 0 | 0 | 42 | 35 | 88 |
| E** | 40 | 81 | 91 | 96 | 97 |
| F | 0 | 27 | 54 | 71 | 93 |
| G | 0 | 13 | 32 | 77 | 97 |
| H | 0 | 44 | 72 | 99 | 98 |
| I | 41 | 58 | 92 | 95 | 100 |
| J | 0 | 0 | 0 | 45 | 91 |
| K* | 0 | 0 | 65 | 95 | 93 |
| L | 0 | 36 | 58 | 93 | 94 |

* Ein Beruhigungsmittel bestehend aus einer Lösung dieser Verbindung wurde in der Patentschrift US-A-4 049 809 beschrieben. Eine solche Lösung ist jedoch für die lokale äusserliche Anwendung ungeeignet.

** Suppositorien, die eine Tranquillizer-Wirkung aufweisen, wurden aus dieser Verbindung bereitet. Siehe DE-A-1 801 523, Beispiel 12.

Experiment 2

Die Verbindung E wird bei 3 verschiedenen Dosierungen im Krotonöl-Entzündungs-Test in der Maus auf ihre topisch entzündungshemmende Wirkung hin geprüft. Ebenfalls getestet werden Hydrocortison, ein bekanntes topisch entzündungshemmendes Corticosteroid, und eine Reihe von bekannten Verbindungen, welche systemisch entzündungshemmend wirken und nicht zur Klasse der Steroide gehören. Die Testresultate (Hemmung in Prozenten) sind in der unten stehenden Tabelle angegeben. Wie man diesen Resultaten entnehmen kann, entfaltet die Verbindung E eine vergleichbare Aktivität wie Hydrocortison, währenddem die anderen Verbindungen, welche nicht zur Klasse der Steroide gehören, in wesentlich höheren Dosierungen verabreicht werden müssen, um eine vergleichbare Hemmung zu bewirken, wie die Verbindung E und Hydrocortison.

Topisch entzündungshemmende Wirkung im Mäuseohr-Krotonöl-Test

| Behandlung mit | Dosis (µg/25 µl) | Hemmung in % |
|---|---|---|
| Verbindung E | 100 | 48 |
| | 200 | 67 |
| | 500 | 91 |
| Hydrocortison | 50 | 53 |
| | 125 | 77 |
| | 250 | 98 |
| Phenylbutazon | 1 250 | 33 |
| | 2 500 | 69 |
| Fenoprofen | 1 250 | 50 |
| | 2 500 | 71 |
| Naproxen | 1 250 | 60 |
| | 2 500 | 87 |
| Acetylsalicylsäure | 1 250 | 28 |
| | 2 500 | 36 |
| | 5 000 | 78 |
| | 10 000 | 88 |

Experiment 3

Die topisch entzündungshemmende Wirkung der Verbindung E wird bei einer Dosierung von 100 µg/0,1 ml im Cantharidin-Entzündungs-Test in der Ratte bestimmt, wobei man adrenalektomierte und intakte Ratten verwendet. Wie man den Testresultaten in der untenstehenden Tabelle entnehmen kann, entfaltet die Verbindung E an der adrenalektomierten Ratte etwa die gleich grosse Wirkung wie an der intakten Ratte. Dies zeigt an, dass der primäre Wirkungsmechanismus nicht in der Stimulierung der Nebennierenrinde besteht.

| | Behandlung* mit | | Hemmung in % |
|---|---|---|---|
| Intakte Ratten | Trägermaterial | $12.0 \pm 0.4$ | |
| | Cantharidin | $25.2 \pm 1.5$ | |
| | Verbindung E + Cantharidin | $18.0 \pm 1.1$ | 55 |
| Adrenalektomierte Ratten | Trägermaterial | $11.4 \pm 0.2$ | |
| | Cantharidin | $25.5 \pm 1.0$ | |
| | Verbindung E + Cantharidin | $18.6 \pm 0.6$ | 49 |

* Verbindung 24 Stunden nach dem Reizmittel verabreicht.

Experiment 4

Die topisch entzündungshemmenden Wirkungen der Verbindung E, Hydrocortison und Indomethacin werden bei verschiedenen Dosierungen im Oxazolon-Entzündungs-Test in vorsensibilisierten Mäusen bestimmt. Die Testresultate (Hemmung in %) sind in der untenstehenden Tabelle angegeben.

| Verbindung | Dosis (µg/10 µl) | Hemmung in % |
|---|---|---|
| Verbindung E | 100 | 51 |
| | 500 | 75 |
| Hydrocortison | 50 | 24 |
| | 200 | 49 |
| Indomethacin | 500 | 32 |
| | 1000 | 59 |

Experiment 5

Die topisch entzündungshemmenden Wirkungen der Verbindung E und Hydrocortison werden durch Verwendung des Baumwollkügelchen-Granuloma-Verfahrens an der Ratte bestimmt. Die in der unten stehenden Tabelle angegebenen Testresultate zeigen, dass die Verbindung E bei Dosierungen von 90 und 270 µg/0,1 ml grösser ist als die Aktivität von Hydrocortison (niedrigere Feucht- und Trockengewichte).

| Behandlung der Kügelchen mit | | Anzahl Kügelchen | durchschnittliches Nettogewicht der Kügelchen in mg feucht | trocken |
|---|---|---|---|---|
| Äthanol (Trägermaterial) | | 20 | 392.5 | 32.6 |
| Hydrocortison | (90 µg/0.1 ml) | 20 | 406.5 | 37.7 |
| | (270 µg/0.1 ml) | 20 | 286.6 | 20.3 |
| Verbindung E | (90 µg/0.1 ml) | 20 | 285.2 | 20.1 |
| | (270 µg/0.1 ml) | 16 | 234.9 | 16.9 |

Experiment 6

Die entzündungshemmende Wirkung der Verbindung E alleine oder in Kombination mit 4-(3-Butoxy-4-methoxybenzyl)-2-imidazolidinon wird im Krotonöl-Test an der Maus bestimmt. Die Verbindung E wird bei verschiedenen Konzentrationen verwendet. 4-(3-Butoxy-4-methoxybenzyl)-2-imidazolidinon wird, sofern vorhanden, in einer Konzentration von 5 Gewichtsprozenten (bezogen auf das Gewicht der applizierten Lösung) eingesetzt. Die Testresultate sind in der untenstehenden Tabelle aufgeführt.

| Verbindung E (µg/0,1 ml) | Gewicht der Ohrstücke in mg | |
|---|---|---|
| | Verbindung E alleine | Verbindung E + 5% 4-(3-Butoxy-4-methoxybenzyl) 2-imidazolidinon |
| 0 | 19,7±0,6 | 17,6±1,0 |
| 100 | 15,9±0,8 | 10,3±0,5 |
| 400 | 11,8±0,6 | 7,4±0,4 |

Trägermaterial (d. h. ohne Krotonöl als Reizmittel): Gewicht der Ohrstücke 7,2± 0,3 mg

Aus der oben stehenden Tabelle geht hervor, dass
4-(3-Butoxy-4-methoxybenzyl)-2-imidazolidinon
in einer Konzentration von 5% die entzündungshemmende Wirkung der Verbindung E potenziert.

Die folgenden Beispiele illustrieren pharmazeutische Präparate, welche eine Verbindung der allgemeinen Formel I enthalten und für die topische Anwendung geeignet sind.

Beispiel A

Eine Crème enthaltend die folgenden Bestandteile, kann in herkömmlicher Weise hergestellt werden:

| Bestandteil | Gewichts-% |
|---|---|
| 8-Chlor-10-(4-methylpiperazino)dibenzo[b,f]thiepin | 0,1-1,00 |
| Glycerin-monostearat S.E. | 10,00 |
| Cetylalkohol | 2,00 |
| Paraffin | 5,00 |
| Methyl-paraben | 0,15 |
| Propyl-paraben | 0,05 |
| Propylenglykol | 20,00 |
| Wasser | auf 100,00 |

In analoger Weise können Crèmes hergestellt werden, welche
8-Chlor-10-(4-methylpiperazino)dibenzo[b,f]-thiepin
als Wirkstoff enthalten.

Beispiel B

Eine Lotion, enthaltend die folgenden Bestandteile, kann in herkömmlicher Weise hergestellt werden:

| Bestandteil | Gewichts-% |
|---|---|
| 8-Chlor-10-(4-methylpiperazino)dibenzo[b,f]thiepin | 0,1-1,00 |
| Cetearylalkohol und Ceteareth-20 | 2,0 |
| Glycerin-monostearat | 4,0 |
| Isopropylmyristat | 10,0 |
| Ceteth-20 | 5,0 |
| Cetylalkohol | 2,0 |
| Dehydroessigsäure | 0,1 |
| 4-(3-Butoxy-4-methoxybenzyl)-2-imidazolidinon | 0,5 |
| Xanthan-gummi | 0,5 |
| Polyäthylenglykol 400 | 5,0 |
| Wasser | auf 100,00 |

In analoger Weise können Lotionen hergestellt werden, welche
8-Chlor-10-(4-methylpiperazino)dibenzo[b,f]-thiepin
als Wirkstoff enthalten.

Beispiel C

Eine Salbe, enthaltend die folgenden Bestandteile, kann in herkömmlicher Weise hergestellt werden:

| Bestandteil | Gewichts-% |
|---|---|
| 8-Chlor-10-(4-methylpiperazino)dibenzo[b,f]thiepin | 0,1-1,00 |
| Hydriertes Lanolin | 20,00 |
| Propylenglykol | 20,00 |
| Mineralöl | 10,00 |
| Microcrystallines Wachs | 2,50 |
| Paraffin | auf 100,00 |

In analoger Weise können Salben hergestellt werden, welche
8-Chlor-10-(4-methylpiperazino)dibenzo[b,f]-thiepin
als Wirkstoff enthalten.

## Patentansprüche

1. Entzündungshemmende Mittel für die lokale äusserliche Anwendung, enthaltend eine wirksame Menge einer Verbindung der allgemeinen Formel

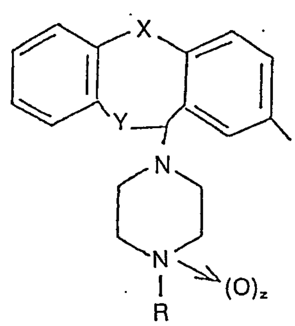

I

worin X ein Schwefel- oder Sauerstoffatom, Y die Gruppe $-CH=$, $-CH_2-$ oder $-N=$, R niederes Alkyl oder Hydroxy-niederes Alkyl, R′ niederes Alkyl, Halogen, Cyano, niederes Alkylthio, Carboxy oder niederes Alkylcarboxy und Z die Zahl 0 oder 1 bedeuten, mit der Massgabe, dass R′ Brom und X ein Schwefelatom bedeuten, wenn Y die Gruppe $-CH_2-$ bedeutet, und X ein Sauerstoffatom bedeutet, wenn Y die Gruppe $-N=$ bedeutet, oder ein pharmazeutisch annehmbares Salz davon und ein für die lokale äusserliche Anwendung bestimmtes pharmazeutisch annehmbares Trägermaterial.

2. Arzneimittel gemäss Anspruch 1, dadurch gekennzeichnet, dass sie als Gele, Crèmes, Lotionen, Salben, Puders oder Aerosole vorliegen.

3. Arzneimittel gemäss Anspruch 1 oder 2, dadurch gekennzeichnet, dass X ein Schwefelatom, Y die Gruppe $-CH=$, R Methyl und Z die Zahl 0 bedeuten.

4. Arzneimittel gemäss Anspruch 1 oder 2, dadurch gekennzeichnet, dass sie als Verbindung der in Anspruch 1 definierten allgemeinen Formel I
8-Chlor-10-(4-methylpiperazino)dibenzo[b,f]-thiepin
enthalten.

5. Arzneimittel gemäss einem der Ansprüche 1–4, dadurch gekennzeichnet, dass sie, bezogen auf das Gesamtgewicht des Arzneimittels, 0,01 bis 10 Gewichtsprozent einer Verbindung der in Anspruch 1 definierten allgemeinen Formel I enthalten.

6. Arzneimittel gemäss Anspruch 5, dadurch gekennzeichnet, dass sie, bezogen auf das Gesamtgewicht des Arzneimittels, 0,1 bis 1,0 Gewichtsprozent einer Verbindung der in Anspruch 1 definierten allgemeinen Formel I enthalten.

7. Arzneimittel enthaltend eine Verbindung der allgemeinen Formel

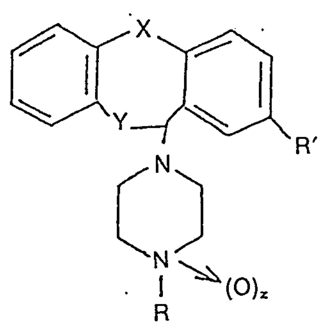

I

worin X ein Schwefel- oder Sauerstoffatom, Y die Gruppe $-CH=$, $-CH_2-$ oder $-N=$, R niederes Alkyl oder Hydroxy-niederes Alkyl, R′ niederes Alkyl, Halogen, Cyano, niederes Alkylthio, Carboxy oder niederes Alkylcarboxy und Z die Zahl 0 oder 1 bedeuten, mit der Massgabe, dass R′ Brom und X ein Schwefelatom bedeuten, wenn Y die Gruppe $-CH_2-$ bedeutet, und X ein Sauerstoffatom bedeutet, wenn Y die Gruppe $-N=$ bedeutet, oder ein pharmazeutisch annehmbares Salz davon und eine Verbindung der allgemeinen Formel

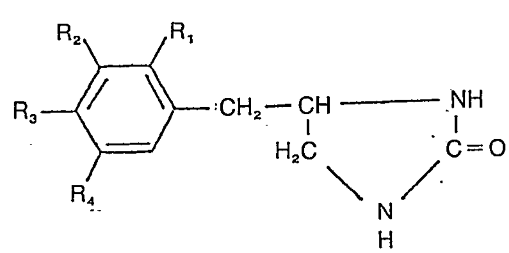

II

worin $R_1$ Halogen, Wasserstoff, niederes Alkyl oder niederes Alkoxy und $R_2$, $R_3$ und $R_4$ unabhängig voneinander Wasserstoff, niederes Alkoxy oder Hydroxy-niederes Alkoxy bedeuten, mit der Massgabe, dass mindestens einer der Reste $R_2$, $R_3$ und $R_4$ einen sauerstoffhaltigen Substituenten bedeutet, oder je zwei benachbarte Reste $R_1$, $R_2$, $R_3$ und $R_4$ zusammen Methylendioxy bedeuten.

8. Arzneimittel gemäss Anspruch 7, dadurch gekennzeichnet, dass X ein Schwefelatom, Y die Gruppe –CH=, R Methyl und Z die Zahl 0 bedeuten.

9. Arzneimittel gemäss Anspruch 7, dadurch gekennzeichnet, dass sie als Verbindung der in Anspruch 7 definierten allgemeinen Formel I
8-Chlor-10-(4-methylpiperazino)-dibenzo[b,f]-thiepin
enthalten.

10. Arzneimittel gemäss einem der Ansprüche 7–9, dadurch gekennzeichnet, dass sie, bezogen auf das Gesamtgewicht des Arzneimittels, 0,01 bis 10 Gewichtsprozent einer Verbindung der in Anspruch 7 definierten allgemeinen Formel I enthalten.

11. Arzneimittel gemäss Anspruch 10, dadurch gekennzeichnet, dass sie, bezogen auf das Gesamtgewicht des Arzneimittels, 0,1 bis 1,0 Gewichtsprozent einer Verbindung der in Anspruch 7 definierten allgemeinen Formel I enthalten.

12. Arzneimittel gemäss einem der Ansprüche 7–11, dadurch gekennzeichnet, dass sie, bezogen auf das Gesamtgewicht des Arzneimittels, bis zu 10 Gewichtsprozent einer Verbindung der in Anspruch 7 definierten allgemeinen Formel II enthalten.

13. Arzneimittel gemäss einem der Ansprüche 7–12, dadurch gekennzeichnet, dass sie als Verbindung der in Anspruch 7 definierten allgemeinen Formel II
4-(3-Butoxy-4-methoxybenzyl)-2-imidazolidinon
enthalten.

14. Verwendung von Verbindungen der in Anspruch 1 definierten allgemeinen Formel I oder von pharmazeutisch annehmbaren Salzen davon, gegebenenfalls in Kombination mit Verbindungen der in Anspruch 7 definierten allgemeinen Formel II, für die Herstellung von Arzneimitteln zur lokalen äusserlichen Behandlung von Entzündungen der Hautgewebe.

15. Verwendung gemäss Anspruch 14, dadurch gekennzeichnet, dass die Verbindung der in Anspruch 1 definierten Formel I
8-Chlor-10-(4-methylpiperazino)dibenzo[b,f]-thiepin
ist.

## Claims

1. Anti-inflammatory compositions for local topical administration containing an effective amount of a compound of the general formula

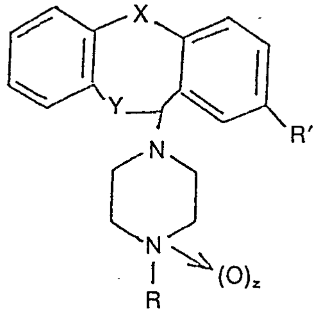

I

wherein X signifies a sulphur or oxygen atom, Y signifies the group –CH=, $-CH_2-$ or –N=, R signifies lower alkyl or hydroxy-lower alkyl, R′ signifies lower alkyl, halogen, cyano, lower alkylthio, carboxy or lower alkylcarboxy and Z signifies the number 0 or 1, with the proviso that R′ signifies bromine and X signifies a sulphur atom when Y signifies the group $-CH_2-$, and X signifies an oxygen atom when Y signifies the group –N=,
or a pharmaceutically acceptable salt thereof and a pharmaceutically acceptable carrier material which is designed for local topical application.

2. Medicaments in accordance with claim 1, characterized in that they are present as gels, creams, lotions, salves, powders or aerosols.

3. Medicaments in accordance with claim 1 or 2, characterized in that X signifies a sulphur atom, Y signifies the group –CH=, R signifies methyl and Z signifies the number 0.

4. Medicaments in accordance with claim 1 or 2, characterized in that they contain
8-chloro-10-(4-methyl-piperazino)dibenzo[b,f]-thiepin
as the compound of general formula I defined in claim 1.

5. Medicaments in accordance with any one of claims 1–4, characterized in that they contain 0.01 to 10 weight percent of a compound of general formula I defined in claim 1 based on the total weight of the medicament.

6. Medicaments in accordance with claim 5, characterized in that they contain 0.1 to 1.0 weight percent of a compound of general formula I defined in claim 1 based on the total weight of the medicament.

7. Medicaments containing a compound of the general formula

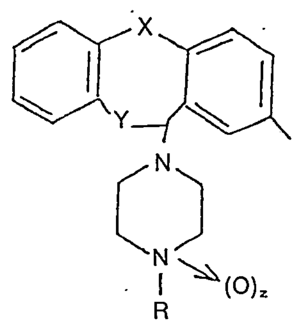

I

wherein X signifies a sulphur or oxygen atom, Y signifies the group –CH=, $-CH_2-$ or –N=, R signifies lower alkyl or hydroxy-lower alkyl, R′ signifies lower alkyl, halogen, cyano, lower alkylthio, carboxy or lower alkylcarboxy and Z signifies the number 0 or 1, with the proviso that R′ signifies bromine and X signifies a sulphur atom when Y signifies the group $-CH_2-$, and X signifies an oxygen atom when Y signifies the group –N=,
or a pharmaceutically acceptable salt thereof and a compound of the general formula

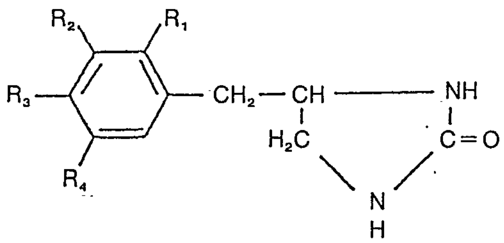

II

wherein $R_1$ signifies halogen, hydrogen, lower alkyl or lower alkoxy and $R_2$, $R_3$ and $R_4$ each independently signify hydrogen, lower alkoxy or hydroxy-lower alkoxy, with the proviso that at least one of the residues $R_2$, $R_3$ and $R_4$ signifies an oxygen-containing substituent, or two adjacent residues $R_1$, $R_2$, $R_3$ and $R_4$ together signify methylenedioxy.

8. Medicaments in accordance with claim 7, characterized in that X signifies a sulphur atom, Y signifies the group $-CH=$, R signifies methyl and Z signifies the number 0.

9. Medicaments in accordance with claim 7, characterized in that they contain
8-chloro-10-(4-methylpiperazino)dibenzo[b,f]-thiepin
as the compound of general formula I defined in claim 7.

10. Medicaments in accordance with any one of claims 7–9, characterized in that they contain 0.01 to 10 weight percent of a compound of general formula I defined in claim 7 based on the total weight of the medicament.

11. Medicaments in accordance with claim 10, characterized in that they contain 0.1 to 1.0 weight percent of a compound of general formula I defined in claim 7 based on the total weight of the medicament.

12. Medicaments in accordance with any one of claims 7–11, characterized in that they contain up to 10 weight percent of a compound of general formula II defined in claim 7 based on the total weight of the medicament.

13. Medicaments in accordance with any one of claims 7–12, characterized in that they contain 4-(3-butoxy-4-methoxybenzyl)-2-imidazolidinone as the compound of general formula II defined in claim 7.

14. The use of compounds of general formula I defined in claim 1 or of pharmaceutically acceptable salts thereof, optionally in combination with compounds of general formula II defined in claim 7, for the manufacture of medicaments for the local topical treatment of inflammation of dermal tissues.

15. The use in accordance with claim 14, characterized in that the compound of formula I defined in claim 1 is
8-chloro-10-(4-methylpiperazino)dibenzo[b,f]-thiepin.

## Revendications

1. Médicaments anti-inflammatoires pour l'application locale externe contenant une quantité efficace d'un composé de formule générale

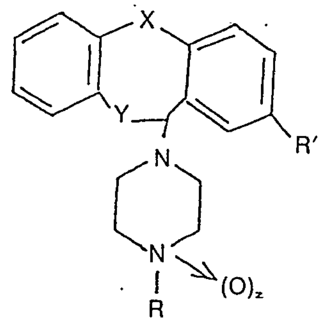

I

dans lequel X est un atome de soufre ou d'oxygène, Y est le groupe $-CH=$, $-CH_2-$ ou $-N=$, R est un alkyle inférieur ou un hydroxy-alkyle inférieur, R' est un alkyle inférieur, halogène, cyano, alkylthio inférieur, carboxy ou alkylcarboxy inférieur et Z est le nombre 0 ou 1 avec la règle que R' représente le brome et X un atome de soufre quand Y représente le groupe $-CH_2-$, et X représente un atome d'oxygène, quand Y représente le groupe $-N=$,
ou un de ses sels pharmaceutiquement acceptables et un matériau véhicule pharmaceutiquement acceptable et adapté à l'application locale externe.

2. Médicaments selon la revendication 1, caractérisés en ce qu'ils se présentent sous forme de gels, crèmes, lotions, onguents, poudres ou aérosols.

3. Médicaments selon la revendication 1 ou 2, caractérisés en ce que X représente un atome de soufre, Y le groupe $-CH=$, R le méthyle et Z le nombre 0.

4. Médicaments selon la revendication 1 ou 2, caractérisés en ce qu'ils contiennent comme composé de formule générale I définie dans la revendication 1, la
8-chloro-10-(4-méthylpipérazino)dibenzo[b,f]-thiépine.

5. Médicaments selon l'une des revendications 1–4, caractérisés en ce qu'ils contiennent, rapporté au poids total du médicament, de 0,01 à 10% en poids d'un composé de formule générale I définie dans la revendication 1.

6. Médicaments selon la revendication 5, caractérisés en ce qu'ils contiennent, rapporté au poids total du médicament, de 0,1 à 1,0% en poids d'un composé de formule générale I définie dans la revendication 1.

7. Médicaments contenant un composé de formule générale

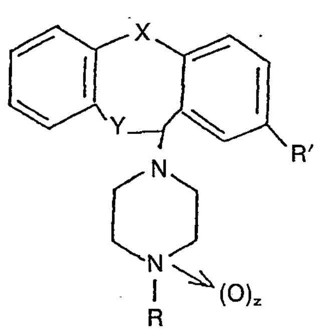

I

dans lequel X est un atome de soufre ou d'oxygène, Y est le groupe $-CH=$, $-CH_2-$ ou $-N=$, R est un alkyle inférieur ou un hydroxy-alkyle inférieur, R′ est un alkyle inférieur, halogène, cyano, alkylthio inférieur, carboxy ou alkylcarboxy inférieur et Z est le nombre 0 ou 1 avec la règle que R′ représente le brome et X un atome de soufre quand Y représente le groupe $-CH_2-$, et X représente un atome d'oxygène, quand Y représente le groupe $-N=$,

ou un de ses sels pharmaceutiquement acceptables et un composé de formule générale

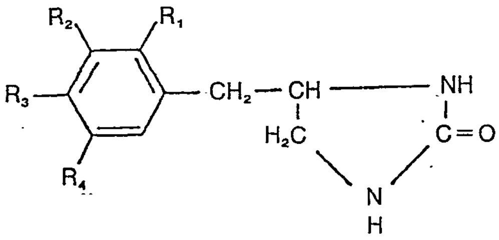

II

dans lequel $R_1$ est un halogène, hydrogène, alkyle inférieur ou alcoxy inférieur et $R_2$, $R_3$ et $R_4$ indépendamment l'un de l'autre, sont un hydrogène, alcoxy inférieur ou hydroxy-alcoxy inférieur, avec la règle qu'au moins l'un des restes $R_2$, $R_3$ et $R_4$ représente un substituant contenant de l'oxygène, ou que chaque couple de deux restes voisins $R_1$, $R_2$, $R_3$ et $R_4$ représentent ensemble le méthylènedioxy.

8. Médicaments selon la revendication 7, caractérisés en ce que X représente un atome de soufre, Y le groupe $-CH=$, R le méthyle et Z le nombre 0.

9. Médicaments selon la revendication 7, caractérisés en ce qu'ils contiennent comme composé de formule générale I définie dans la revendication 7, la

8-chloro-10-(4-méthylpipérazino)dibenzo[b,f]-thiépine.

10. Médicaments selon l'une des revendications 7–9, caractérisés en ce qu'ils contiennent, rapporté au poids total du médicament, de 0,01 à 10% en poids d'un composé de formule générale I définie dans la revendication 7.

11. Médicaments selon la revendication 10, caractérisés en ce qu'ils contiennent, rapporté au poids total du médicament, de 0,1 à 1,0% en poids d'un composé de formule générale I définie dans la revendication 7.

12. Médicaments selon l'une des revendications 7–11, caractérisés en ce qu'ils contiennent, rapporté au poids totale du médicament, jusqu'à 10% en poids d'un composé de formule générale II définie dans la revendication 7.

13. Médicaments selon l'une des revendications 7–12, caractérisés en ce qu'ils contiennent, comme composé de formule générale II définie dans la revendication 7 de la

4-(3-butoxy-4-méthoxybenzyl)-2-imidazolidinone.

14. Utilisation de composés de formule générale 1 définie dans la revendication I ou de leurs sels pharmaceutiquement acceptables, le cas échéant en combinaison avec des composés de la formule générale II définie dans la revendication 7 pour la production de médicaments destinés au traitement externe local d'inflammations du tissu de la peau.

15. Utilisation selon la revendication 14, caractérisée en ce que le composé de formule I définie dans la revendication 1 est la

8-chloro-10-(4-méthylpipérazino)dibenzo-[b,f]thiépine.